# EUROPEAN PATENT APPLICATION

(11) **EP 4 763 233 A1**
(43) Date of publication of application: **24.06.2026**
(21) Application number: 25222192.4
(22) Date of filing: 10.12.2025
(51) Int. Cl.: A61L 9/12

(54) **DEVICE FOR THE CONTROLLED DELIVERY OF ODOURS AND DELIVERY MANIFOLD FOR SUCH DEVICE**

(30) Priority: 19.12.2024 IT 202400029013
(71) Applicant: CyNexo S.r.l., 33050 Trivignano Udinese (UD) (IT)
(72) Inventor: PARUSSO, Stefano, 33030 Campoformido (UD) (IT); CASTLETON, Christopher John Stirling, 33030 Camino al Tagliamento (UD) (IT)
(74) Representative: Frare, Paolo

(57) **Abstract**

The present finding relates to a device (1) for the controlled delivery of odours, comprising a delivery manifold (7) for such device (1).

Such delivery manifold (7) comprises: a central body (8) crossed by one or more main ducts (9); a plurality of peripheral ducts (20), each of which puts one of said one or more main ducts (9) in fluid communication with the outside of the central body (8); a plurality of tubular cartridges (21) provided with a transit duct (22) passing through them from a suction end (23) of them to a delivery end (24) of them, wherein the delivery end (24) of each of such tubular cartridges (21) is fixed or fixable in a hermetic way, in a removable manner, to one of the peripheral ducts (20), so as to put the respective transit duct (22) in fluid communication with such peripheral duct (20), wherein each of the tubular cartridges (21) contains a non-return valve (31) positioned in the transit duct (22) to prevent the passage of a fluid through the transit duct (22) from the delivery end (24) to the suction end (23).

## Description

The present finding relates to a device for the controlled delivery of odours and to a delivery manifold for such device.

Nowadays, devices adapted to selectively deliver one or more odours are known, for example in order to carry out scientific studies and/or clinical trials on the effect of such odours on individuals and/or to achieve desired physical or psychological effects on individuals, through the controlled administration of specific odours.

A known type of such devices comprises a plurality of containers, inside which an odorous substance can be deposited, and pumping or blowing means, for example one or more piezoelectric pumps, configured to selectively introduce pressurized air into each container through an inlet opening thereof, typically provided with a non-return valve, and then to cause the release of such air from the container, carrying the odour generated by the saturation, in the volume available inside the container, of the odorous substance contained therein, through an outlet opening of the container connected, by means of appropriate piping and non-return valves, to a delivery manifold. The delivery manifold is crossed by a main duct in which, in use, a constant air flow is made to pass, which is then conveyed, by means of suitable piping, to an outlet opening of the device, to which a straw or other piping can be applied, adapted to allow the air flow to be conveyed to the user's nose.

Such known devices further comprise an electronic control device (for example an electronic board provided with a microcontroller), typically interfaced with a computer and configured to control the various electrical and electronic components of the device.

In use, by selectively activating the pumping or blowing means of the device, the odours generated by the odorous substances contained in one or more of the containers are selectively introduced into the delivery manifold, where they mix with the air flow transiting therethrough, and are then conveyed, together with such air flow, to the outlet opening of the device, so that they can be brought to the user's nose, for example through a straw.

An example of such a known type of device, adapted to deliver odours in a controlled manner, is produced by the Applicant and marketed under the commercial name "Sniff-nano".

The delivery manifold of such known-type devices is typically made of a central body crossed by the main duct in which, in use, the constant air flow is made to transit and is then sent to the outlet opening of the device.

On the lateral wall of the central body, there are peripheral ducts that put the main duct in fluid communication with the outside of the delivery manifold; inside such peripheral ducts, an equal number of non-return valves are pressure-fitted, which prevent the odours from the various containers, entering the main duct through the peripheral ducts, from flowing back towards the respective containers.

At the end of each peripheral duct facing the outside of the delivery manifold, an engaging element is further fixed, adapted to fix and hydraulically connect the respective peripheral duct with one end of a pipe adapted to put the respective peripheral duct in fluid communication with the outlet opening of one of the containers.

Although such known solutions have numerous advantages, they nevertheless have some room for improvement.

In particular, it has been found that the non-return valves present in the peripheral ducts of the delivery manifold are subject to accumulation of odour residues; therefore, if one wishes to use a peripheral duct, previously used for the delivery of a different odour, for the delivery of a given odour, it is first necessary to remove and clean or replace the non-return valve contained in such peripheral duct.

However, the removal of the non-return valves from the respective peripheral ducts proves to be rather inconvenient and complex, requiring, after the removal of the respective engaging element from a peripheral duct, access to the inside of such peripheral duct by a tool with an elongated and narrow shape (which cannot always be readily available), capable of engaging the non-return valve contained in the peripheral duct and exerting sufficient traction on it to extract it from the latter.

The main task of the present finding is to overcome the above-mentioned drawback, and in particular to obtain a device for the controlled delivery of odours and a delivery manifold for such device, which allow a quick and easy removal, and quick and easy positioning, of the non-return valves that connect the containers of odorous substances to the delivery manifold.

Within the scope of such task, an object of the present finding is to obtain a device for the controlled delivery of odours and a delivery manifold for such device, which can be easily used both in the field of fundamental research and in clinical settings.

The task and the object according to the present finding are achieved by making a device for the controlled delivery of odours as in claim 1.

The task and the object according to the present finding are also achieved by making a delivery manifold for a device for the controlled delivery of odours as in claim 14.

The features and advantages of the present finding will become more apparent from the following description, provided by way of example and not limitation, with reference to the attached schematic drawings, in which:
- Figure 1 is a schematic view of some components of a device according to the finding;
- Figure 2 is a perspective view of an advantageous embodiment of the delivery manifold and of a container of a device according to the finding;
- Figure 3 is an exploded perspective view of the delivery manifold of Figure 2;
- Figure 4 is a perspective view of a component of the delivery manifold of Figure 3;
- Figure 5 is a sectional view of the component of Figure 4;
- Figures 6 and 7 are respectively a perspective view and a side view of a part of the delivery manifold of Figure 2;
- Figure 8 is a sectional view taken according to plane VIII-VIII of Figure 7;
- Figure 9 is an exploded perspective view of a component of the delivery manifold of Figure 2;
- Figure 10 is a sectional view taken according to a longitudinal plane of Figure 9;
- Figure 11 is a side view of a component of the delivery manifold of Figure 2;
- Figure 12 is a sectional view taken according to a longitudinal plane of the component of Figure 11;
- Figure 13 is an exploded perspective view according to a further advantageous embodiment of a delivery manifold of a device according to the finding;
- Figures 14 and 15 are perspective views of a component of the delivery manifold of Figure 13;
- Figure 16 is a side view of the component of Figures 14 and 15, with some parts depicted in transparency;
- Figure 17 is a sectional view taken according to a longitudinal plane of the component of Figures 14 to 16;
- Figure 18 is an enlarged detail of Figure 16;
- Figure 19 is a perspective view of a further embodiment of a delivery manifold of a device according to the finding;
- Figure 20 is an exploded perspective view of the delivery manifold of Figure 19;
- Figure 21 is a first side view of a component of the delivery manifold of Figures 19 and 20;
- Figure 22 is a second side view, from a different angle, of the component of Figure 21, with some parts depicted in transparency;
- Figure 23 is a bottom view of Figure 21;
- Figure 24 is a top view of Figure 21
- Figure 25 is a perspective view of a further advantageous embodiment of a delivery manifold of a device according to the finding.

With reference to the attached figures, an advantageous embodiment of a device for the controlled delivery of odours according to the finding is generally indicated with the reference number 1.

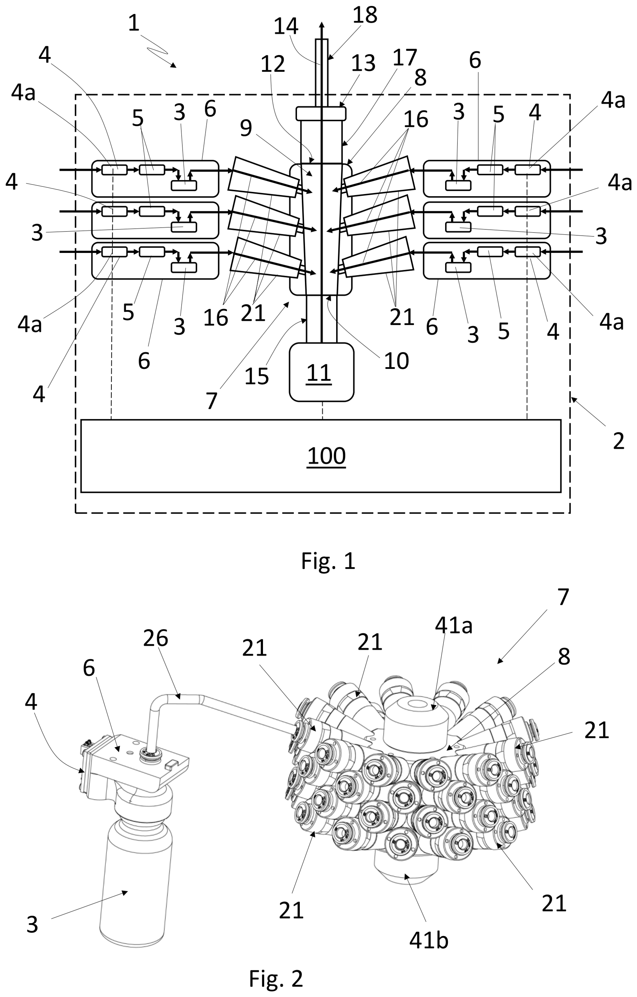

Preferably, the device 1 comprises a containing and/or supporting body 2 (schematically indicated in Figure 1 as a dashed rectangle), for example, but not necessarily, box-shaped, to which the various components of the device 1 are removably fixed, either directly or indirectly.

The device 1 further comprises electronic control means 100 (for example, an electronic board, a microprocessor, a microcontroller, etc.) configured to control the various electrical and electronic components of the device 1.

Advantageously, such electronic control means 100 are operatively connected, or configured to be operatively connected, to a processing system, such as for example a computer, not illustrated, outside the device 1 and advantageously configured to program and/or control such electronic control means 100.

The device 1 comprises a plurality of containers 3, inside which an odorous substance, not illustrated, can be deposited.

It is noted that the expression "odorous substance" refers to a substance, for example, solid, liquid, or gaseous, capable of emitting an odour, for example a perfumed liquid, an essence, or any substance that generates an odour, etc.

The containers 3 can be, for example, small jars or other box-like bodies, for example made of plastic or glass.

Such containers 3 are preferably capable of being hermetically opened and reclosed, so as to allow the insertion and/or removal of an odorous substance, for example to replace it or to add new substance.

In other advantageous embodiments, such containers 3 are not openable, in which case, they are intended for a so-called single use, or they must be completely replaced when the odorous substance contained therein is fully consumed and/or is no longer capable of emitting the respective odour.

The containers 3 further have an inlet opening and an outlet opening, not illustrated, respectively adapted to allow the entry and exit of a fluid (for example air or air mixed with an odour).

It is noted that, according to the finding, the inlet and outlet openings of the container 3 can be two completely separate openings (for example, they can be two distinct holes), or, in other advantageous embodiments, they can be two distinct portions of a same opening; for example, the outlet opening can be a central portion of a hole, not illustrated, advantageously having a circular plan, into which a pipe 26 is positioned or positionable, from which a fluid exits, and the inlet portion can be, for example, an annular portion of the same hole, which surrounds the first portion, through which the fluid enters the container 3.

The device 1 further comprises first pumping or blowing means 4 configured to selectively introduce pressurized air into the containers 3 (through the unillustrated inlet opening thereof) and subsequently to cause the release of such air from the respective containers 3 (through the unillustrated outlet opening thereof). Such pumping or blowing means 4 are advantageously controlled by the electronic control means 100. Advantageously, the first pumping or blowing means 4 are hydraulically connected to the containers 3 through inlet valves 5 of the so-called "non-return" type, so as to prevent backflow of air from the containers 3 towards the first pumping or blowing means 4.

In advantageous embodiments, such first pumping or blowing means 4 advantageously comprise a plurality (preferably one for each container 3) of pumps 4a, preferably of the piezoelectric type, configured to selectively introduce air into a respective container 3 (through its unillustrated inlet opening), advantageously causing such air to pass through a respective inlet valve 5 of the so-called "non-return" type, which prevents the backflow of such air from the respective container 3 towards the respective pump 4a. In advantageous embodiments, such as the one schematically illustrated in Figure 1, a container 3, a respective pump 4a, and a respective inlet valve 5 are housed in a respective casing or containing body 6, schematically illustrated in Figure 1 as a rectangle with rounded corners.

In other advantageous embodiments, such as for example that illustrated in Figure 2, a pump 4a and a respective inlet valve 5 (not visible in Figure 2) are housed in a respective casing or containing body 6, and the respective container 3 is fixed to said casing or containing body 6, protruding at least partially outside it.

In other advantageous embodiments, not illustrated, the pumping or blowing means 4 comprise a single pump (or alternatively, multiple pumps parallel to each other), not illustrated, which provides pressurized air to an inlet manifold, not illustrated, provided with a plurality of outlets in turn hydraulically connected, through inlet valves 5 of the so-called "non-return" type, to the inlet openings of respective containers 3. The device 1 further comprises a delivery manifold 7 comprising a central body 8 crossed by one or more main ducts 9, which will be described in greater detail below, an inlet end 10 of which is in fluid communication with second air pumping or blowing means 11, and an outlet end 12 of which is in fluid communication with means for delivering 13 air outside the device 1.

Advantageously, the one or more main ducts 9 are rectilinear, preferably with a circular or polygonal cross-section.

In advantageous embodiments, the second pumping or blowing means 11 comprise a pump, for example of the volumetric type.

Advantageously, the second pumping or blowing means 11 are controlled by the electronic control means 100.

In advantageous embodiments, the second pumping or blowing means 11 are adapted to generate a main air flow, schematically illustrated in Figure 1 with an arrow 14, preferably with a constant flow rate, which is sent to the inlet end 10 of the one or more main ducts 9 through dedicated first hydraulic connection means, such as for example specific first piping 15. Advantageously, such main air flow 14, enriched, as will be explained later, by a desired number of further odorous air flows 16 from respective containers 3, then is released from the outlet end 12 of the one or more main ducts 9 and is conveyed, through second hydraulic connection means, such as for example specific second piping 17, to the means for delivering 13 air outside the device 1, such as for example one or more straws or cannulas 18.

In advantageous embodiments, the one or more main ducts 9 have a cylindrical, truncated-conical, truncated-pyramidal, or prismatic shape.

The central body 8 further comprises a plurality of peripheral ducts 20, which will be described in more detail below, each of which puts one of the one or more main ducts 9 in fluid communication with the outside of the central body 8.

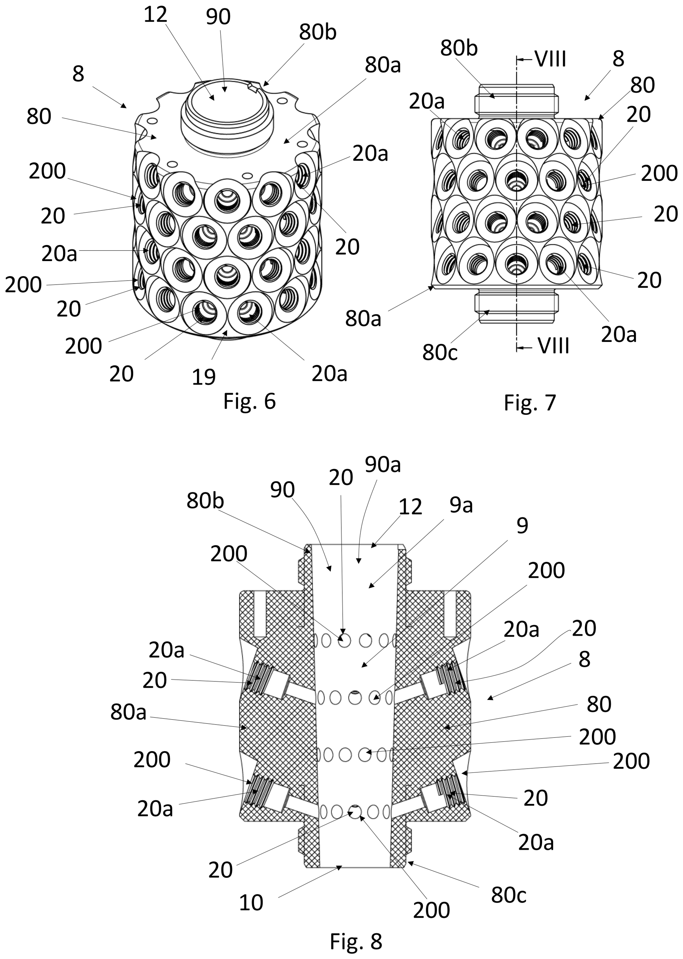

In advantageous embodiments, such as those illustrated in the attached figures, the one or more main ducts 9 extend parallel to a rectilinear longitudinal direction; in advantageous embodiments, such as those illustrated in Figures 1 to 8, each of the peripheral ducts 20 extends along an inclined direction (i.e., neither parallel nor orthogonal) with respect to such rectilinear longitudinal direction. Such specific extension inclination of the peripheral ducts 20 ensures that, in the case where two peripheral ducts 20 open into the internal lateral surface 9a of the same main duct 9 in diametrically opposite positions, thus facing each other, the odorous air flow 16 exiting one of such peripheral ducts 20 is not directed towards the peripheral duct 20 diametrically opposite to it and therefore does not enter it, which would otherwise obstruct the release of air therefrom.

In advantageous embodiments, such as those illustrated in the attached figures, the inlets (i.e., the access openings of the peripheral ducts 20 facing the outside of the central body 8) of the peripheral ducts 20 are arranged on the central body 8 at a plurality of overlapping levels.

Advantageously, the inlets of the peripheral ducts 20 on one of such levels are placed side by side and staggered with respect to the inlets of the peripheral ducts 20 on a contiguous level. Such arrangement of the peripheral ducts 20 is particularly advantageous, as it allows to have a high number of peripheral ducts 20 contiguous to each other.

Advantageously, the peripheral ducts 20 have a cross-section that decreases, preferably in a discontinuous manner (i.e., with a succession of sections, each being substantially constant in cross-section and smaller than the previous section), as they proceed from the outside to the inside of the central body 8.

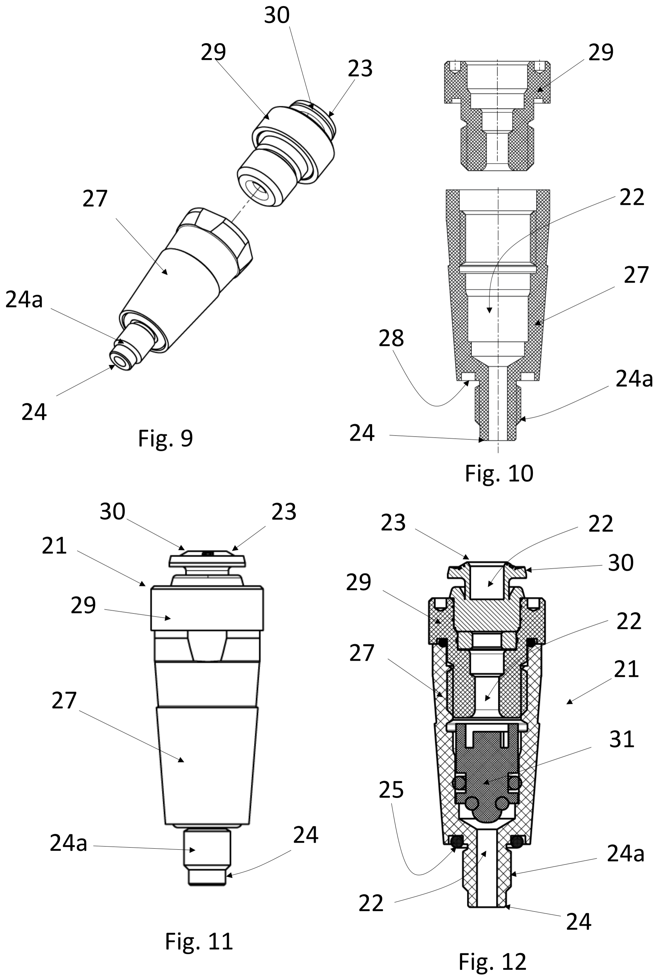

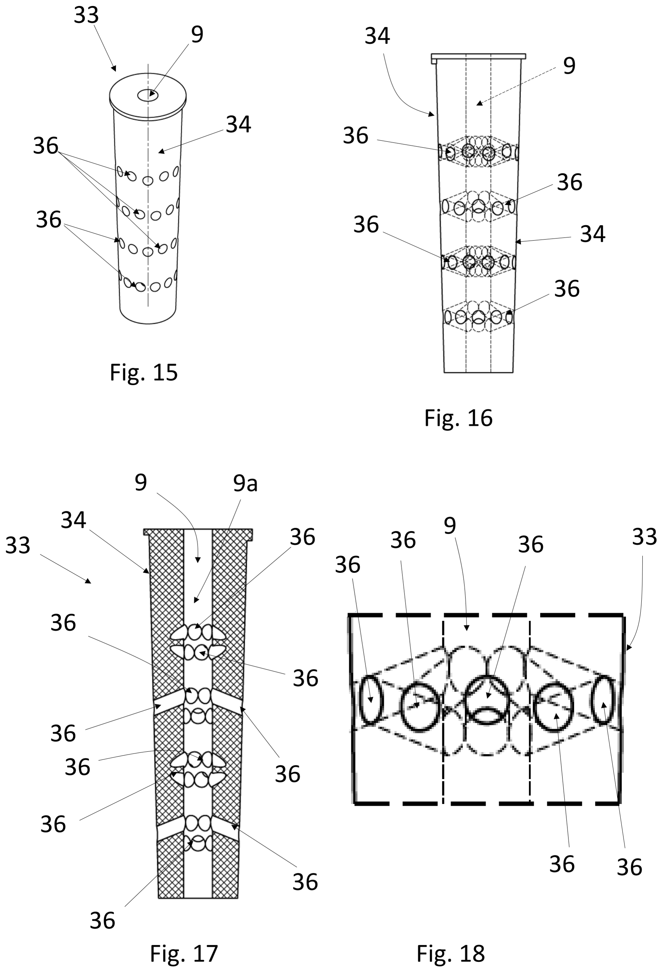

In advantageous embodiments, such as those illustrated in Figures 1 to 18, the central body 8 comprises a single main duct 9.

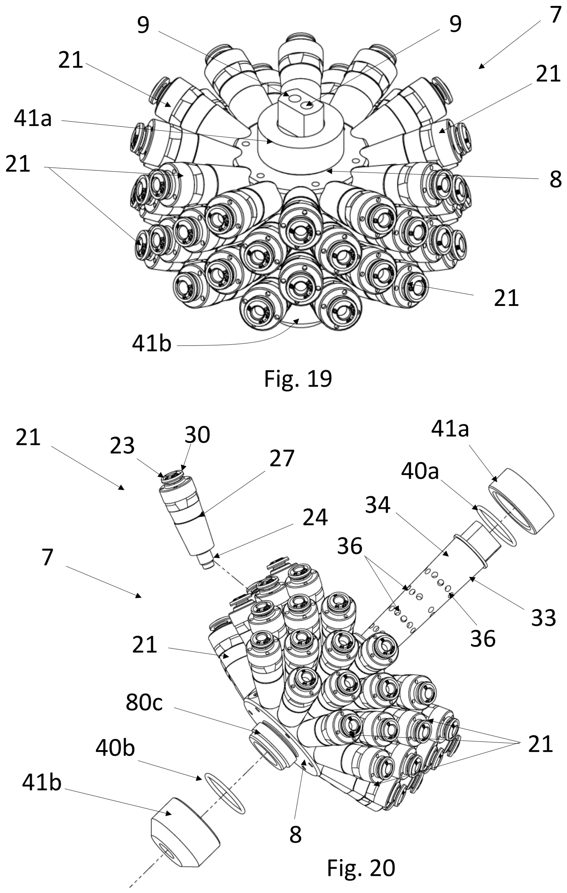

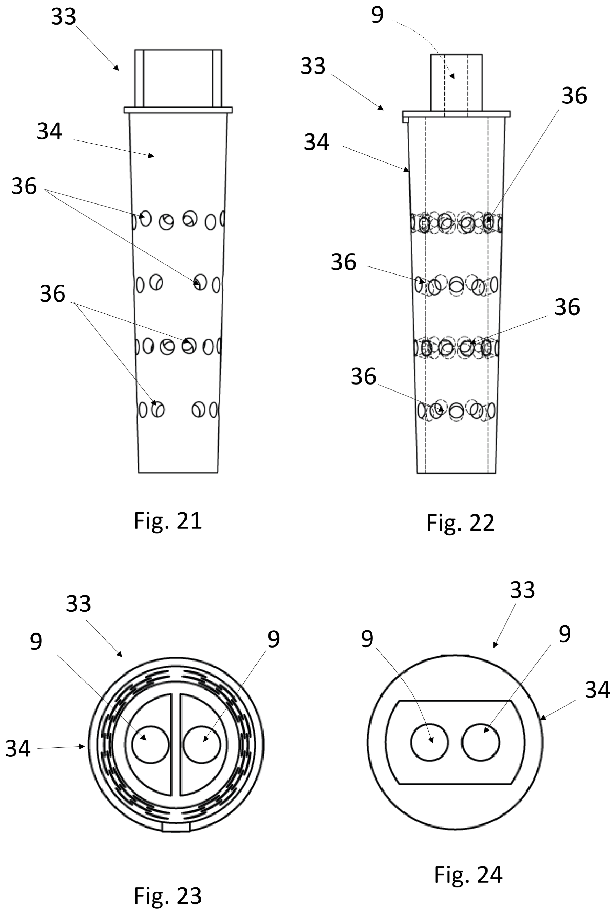

In other advantageous embodiments, such as for example those illustrated in Figures 19 to 25, the central body 8 comprises two main ducts 9, separated and parallel to each other, preferably identical to each other, more preferably rectilinear and with a circular or polygonal cross-section.

In advantageous embodiments, such as those illustrated in the attached figures, the central body 8 comprises a tubular body 80 in which one or more main through holes 90 are obtained, from which a plurality of first peripheral through holes 200 branch off.

In advantageous embodiments, such as for example that illustrated in Figures 1 to 12, the one or more through holes 90 coincide with the one or more main ducts 9 (and the internal lateral surface 90a of such one or more through holes 90 therefore coincides with the internal lateral surface 9a of the one or more main ducts 9), and the first peripheral through holes 200 coincide with the peripheral ducts 20.

In advantageous embodiments, such as those illustrated in the attached figures, the tubular body 80 has a central section 80a, advantageously of cylindrical or prismatic shape, and two distal sections 80b and 80c, also preferably of cylindrical or prismatic shape, but with a smaller plan extension than the central section 80a.

Advantageously, respective ring nuts 41a and 41b are removably fixed, preferably in a removable manner, more preferably with the interposition of suitable annular gaskets 40a and 40b, to the distal sections 80b and 80c.

Advantageously, the ring nuts 41a and 41b are made of plastic material, for example polytetrafluoroethylene (PTFE), or the material known as POM-C.

It is noted that polytetrafluoroethylene (PTFE) and the material known as POM-C are particularly advantageous for these applications, since such materials, due to their specific chemical-physical structure, prevent adhesion of other materials to them and therefore, in particular, of the odorous substances that can be delivered with the device 1.

However, according to the finding, the ring nuts 41a and 41b can also be made of other materials (for example, metal, recycled and/or recyclable plastic, etc.) or combinations of materials.

Other shapes of the tubular body 80, for example cylindrical, prismatic, spherical, cubic, etc., are however contemplated in the finding.

Advantageously, the tubular body 80 is made of plastic material, for example polytetrafluoroethylene (PTFE), or the material known as POM-C, or of metal, or of recycled and/or recyclable plastic, or of a combination of one or more of such materials.

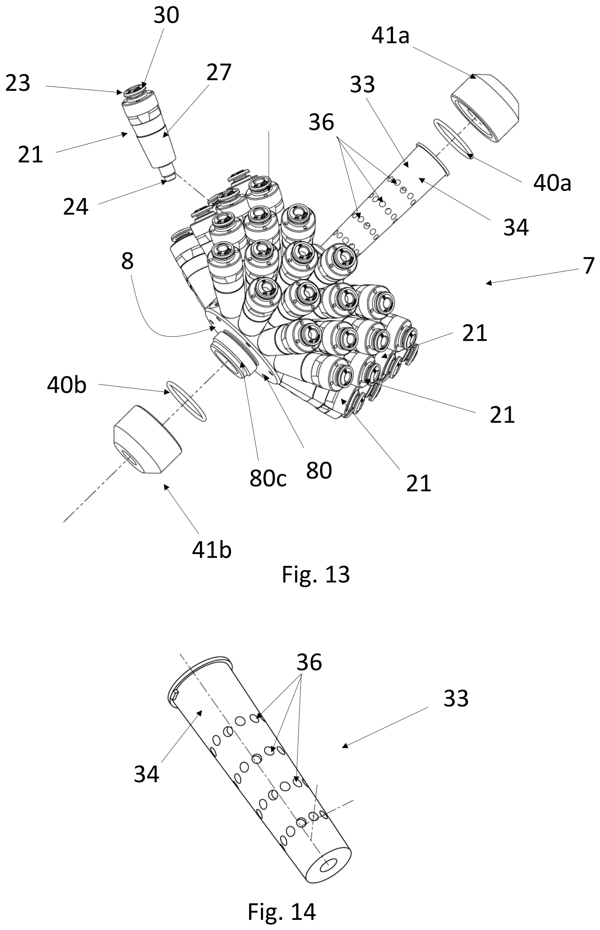

In other advantageous embodiments, such as for example those illustrated in Figures 13 to 25, the central body 8 comprises, in addition to the tubular body 80, one or more tubular inserts 33, whose external lateral surface 34 is at least partially counter-shaped to the internal lateral surface 90a of a respective one of the one or more main through holes 90.

Advantageously, the one or more inserts 33 are made of plastic material, for example polytetrafluoroethylene (PTFE), or the material known as POM-C, or of metal, or of recycled and/or recyclable plastic, or of a combination of one or more of such materials. Advantageously, the one or more tubular inserts 33 are removably inserted into respective one or more main through holes 90 along their entire longitudinal extension.

In advantageous embodiments, such as for example those illustrated in Figures 13 to 25, the one or more main ducts 9 are obtained in one or more of said one or more tubular inserts 33.

Advantageously, the one or more tubular inserts 33 comprise a plurality of second peripheral through holes 36, each of which branches off from one of the one or more main ducts 9 and faces a respective first peripheral through hole 200, together defining one of the peripheral ducts 20.

Advantageously, the central body 8 comprises centering means, not illustrated, for the one or more tubular inserts 33 within the respective one or more main ducts 9 (such as for example dedicated notches or centering chamfers, not illustrated, obtained in the one or more main through holes 90 and/or in the respective one or more tubular inserts 33, in which dedicated centering tabs, not illustrated, are positioned, protruding respectively from the one or more tubular inserts 33 and/or from the one or more main through holes 90), adapted to ensure the correct alignment of the second peripheral through holes 36 with respect to the first peripheral through holes 200.

Advantageously, the inlet openings of the second peripheral through holes 36 are arranged on the respective tubular insert 33 on a plurality of overlapping levels; preferably, the inlet openings of the second peripheral through holes 36 on a same level extend inside the respective tubular insert 33 along inclined directions (or neither parallel nor orthogonal) with respect to the longitudinal direction of the respective tubular insert 33, and such that the second peripheral through holes 36 of each pair of peripheral through holes 36 contiguous to each other extend inside the respective tubular insert 33 in opposite inclined directions.

Advantageously, the one or more tubular inserts 33 are fixed or fixable in a main duct 9 by means of removable coupling means.

In advantageous embodiments, such as for example those illustrated in Figures 13 to 25, such removable coupling means comprise the ring nuts 41a and 41b, which, being fixed to the respective distal section 80b or 80c, keep the one or more tubular inserts 33 in the respective main duct 9.

In other advantageous embodiments, not illustrated, such removable coupling means comprise bayonet coupling means, or screw coupling means, or snap-fit coupling means, etc.

In advantageous embodiments, such as for example those illustrated in Figures 13 to 25, the tubular body 80 comprises a single main through hole 90, advantageously of truncated-conical shape (but it could also be truncated-pyramidal, cylindrical, or prismatic), in which a single tubular insert 33 is inserted, preferably in a removable manner.

In advantageous embodiments, such as for example those illustrated in Figures 13 to 18, such single tubular insert 33 is crossed, along its entire longitudinal extension, by a single main duct 9 (advantageously rectilinear and with a circular or polygonal cross-section), which communicates with the second peripheral through holes 36 of the tubular insert 33, which in turn face corresponding first peripheral through holes 200 of the tubular body 80, together defining the peripheral ducts 20.

In other advantageous embodiments, such as for example those illustrated in Figures 19 to 25, the single tubular insert 33 is crossed, along its entire longitudinal extension, by two main ducts 9, separated and parallel to each other, preferably identical to each other, more preferably rectilinear and with a circular or polygonal cross-section, each of which communicates with a plurality of distinct second peripheral through holes 36 of the tubular insert 33, which in turn face corresponding first peripheral through holes 200 of the tubular body 80, together defining the peripheral ducts 20.

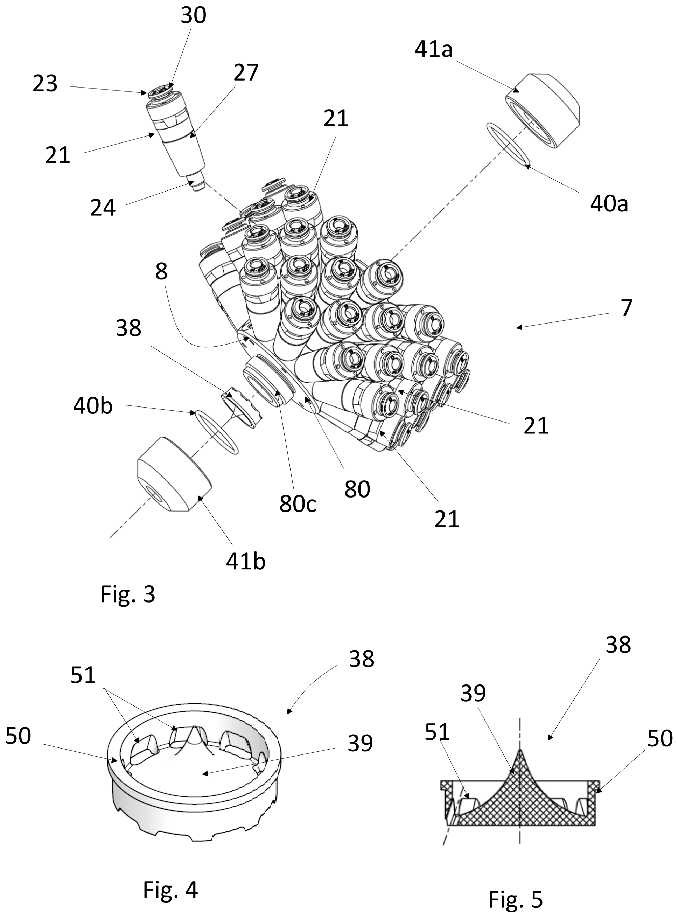

In advantageous embodiments, such as for example those illustrated in Figures 3 to 5, the delivery manifold 7 comprises flow deviating means 38 associated with the main duct 9, to direct a main air flow 14, entering such one or more main ducts 9 through their inlet end 10, towards the internal lateral surface 9a of the one or more main ducts 9.

Advantageously, such flow deviating means 38 can comprise a central element 39, preferably substantially truncated-conical or truncated-pyramidal, more preferably with an arcuate and recessed lateral surface, and preferably with a circular or polygonal plan, fixable to the inlet end 10 of the one or more main ducts 9 and preferably counter-shaped, in plan, thereto, from the peripheral edge of which an annular crown 50 in which a plurality of air passages 51 are obtained protrudes.

Advantageously, such central element 39 is kept fixed to the central body 8 by the ring nut 41b.

The main airflow 14 from the second pumping or blowing means 11 through the first piping 15 impacts the central element 39 at the inlet end 10 and crosses the passages 51, being deviated towards the internal lateral surface 9a of the one or more main ducts 9; in this way, the main air flow 14 flows along the internal lateral surface 9a, effectively removing any odorous substances present therein.

The delivery manifold 7 further comprises a plurality of tubular cartridges 21 provided with a transit duct 22 that passes through them from a suction end 23 of them to a delivery end 24 of them.

Such delivery end 24 is fixed or fixable in a sealed removable manner to one of the peripheral ducts 20, so as to put the respective transit duct 22 in fluid communication with said peripheral duct 20.

In advantageous embodiments, such as for example those illustrated in the attached figures, the peripheral ducts 20 have an internal section 20a, cylindrical, prismatic, truncated-conical, or truncated-pyramidal, and the tubular cartridges 21 have, in correspondence of their delivery end 24, an external lateral surface 24a at least partially counter-shaped to the internal section 20a of the peripheral ducts 20, so as to allow insertion into such internal section 20a.

In an advantageous embodiment, the internal section 20a of the peripheral ducts 20 is cylindrical or threaded truncated-conical, and the external lateral surface 24a of the delivery end 24 is likewise cylindrical or conical and counter-threaded to such internal section 20a, allowing the delivery end 24 to be screwed into the internal section 20a.

In other advantageous embodiments, the delivery end 24 can be fixed to the internal section 20a by means of other known fixing means, such as for example bayonet fixing means, snap-fit fixing means, interlocking fixing means, etc.

Advantageously, the tubular cartridges 21 can be removed from the respective peripheral ducts 20 by gripping them manually or with the aid of dedicated tools, not illustrated, such as for example pliers or a specific tool, not illustrated, fixable to the suction end 23 of such tubular cartridges 21.

Advantageously, the tubular cartridges 21 comprise sealing means, such as for example gaskets 25 protruding from the external surface of the tubular cartridges themselves and positioned so as to engage with the central body 8 when a tubular cartridge 21 is fixed in a peripheral duct 20, thereby preventing the release of fluid from the coupling region between the tubular cartridge 21 and the respective peripheral duct 20.

The transit duct 22 of the tubular cartridges 21 is hydraulically connected, or connectable, in correspondence of the suction end 23, to a pipe 26 that is hydraulically connected, or connectable, to one of the containers 3 (in particular, to its unillustrated outlet opening).

Advantageously, the tubular cartridges 21 have an elongated shape and, preferably, at least one main section 27 advantageously of cylindrical, prismatic, truncated-conical, or truncated-pyramidal shape. Advantageously, the main section 27 is internally hollow and is longitudinally crossed by the transit duct 22.

In advantageous embodiments, such as for example those illustrated in the accompanying figures, the delivery end 24 projects longitudinally from the main section 27 (in the case where such main section 27 is truncated-conical or truncated-pyramidal, it protrudes from the smaller base of the truncated cone or pyramid), coaxially with it, and has a smaller cross-section, thereby defining, in correspondence of the intersection between main section 27 and delivery end 24, an abutment 28 in which a gasket 25 is preferably arranged, for example of the so-called O-ring type.

In other advantageous embodiments, not illustrated, the delivery end 24 is defined by an end of the main section 27 itself.

In advantageous embodiments, such as those illustrated in the attached figures, the end of the main section 27 opposite to the delivery end 24 is open and is hermetically sealed, preferably but not necessarily removably, by a cap 29, which is itself longitudinally crossed by the transit duct 22 and provided with sealing fixing means 30 to an end of a pipe 26 that is hydraulically connected or connectable to one of the containers 3 (in particular to the unillustrated outlet opening thereof).

Advantageously, the sealing fixing means 30 can comprise known-type quick press-fit hydraulic or pneumatic engaging or disengaging means or push-pull hydraulic or pneumatic engaging or disengaging means. Preferably, in the case where the main section 27 has a truncated-conical or truncated-pyramidal shape, the smaller base of such main section faces the delivery end 24; such specific shape is particularly advantageous because it allows the reduction of the size of the peripheral ducts 20 (which must be coupled with the tubular cartridges 21 in correspondence of their delivery end 24), and consequently allows a very high number of peripheral ducts 20 to be in the central body 8 (which corresponds to the possibility of simultaneously introducing a high number of odours into the main duct 9), while still providing the tubular cartridge 21 with a greater transverse extension in correspondence of its suction end 23, suitable for supporting the sealing fixing means 30.

According to the finding, each of the tubular cartridges 21 contains a non-return valve 31 positioned in the transit duct 22 to prevent the passage of a fluid through such transit duct 22 from the delivery end 24 to the suction end 23.

Advantageously, the main section 27 and the cap 29 are made of plastic material, such as polytetrafluoroethylene (PTFE), or the material known as POM-C, or of metal, or of recycled and/or recyclable plastic, or a combination of one or more of such materials.

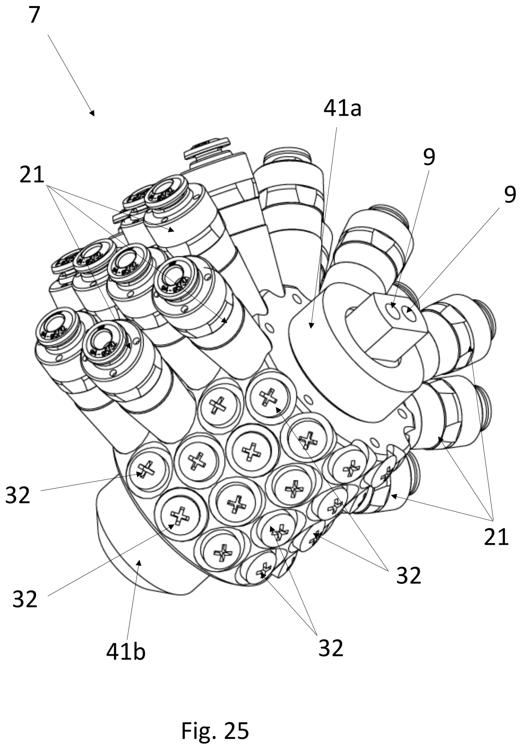

In advantageous embodiments, such as that illustrated in Figure 25, the delivery manifold 7 comprises, in addition to the tubular cartridges 21, one or more closing elements 32 placed to hermetically seal, preferably, but not necessarily, in a removable manner, an equal number of desired peripheral ducts 20, thereby obtaining a desired configuration for the delivery manifold 7.

The operation of the device 1 according to the finding is described below.

After hydraulically connecting (by means of respective pipes 26) the outlet opening of the various containers 3 containing the desired odorous substances to respective tubular cartridges 21 of the delivery manifold 7, it is possible to activate, by means of the electronic control means 100 (possibly controlled in turn by an external computer, not illustrated), the second pumping or blowing means 11, so as to generate a main air flow 14, preferably constant, which crosses the one or more main ducts 9 of the delivery manifold 7.

By then activating the first pumping or blowing means 4, for example one or more of the first pumps 4a, it is possible to generate air flows that enter respective containers 3 through their inlet opening, draw part of the odours contained therein, and then are released from such containers 3 through their outlet opening, being then sent through the respective pipes 26 to respective tubular cartridges 21 in the form of odorous airflows 16; each of such odorous air flows 16 is then introduced into a peripheral duct 20, from which it passes into a respective main duct 9, where it mixes with the main air flow 14 transiting therein. The main air flow 14, enriched with one or more desired odorous air flows 16, then is released from the delivering means 13, such as for example one or more straws or cannulas 18, so as to be sent to a person's nose.

In the case where the main ducts 9 are two, the two main air flows 14, enriched with one or more desired odorous air flows 16, which are released from such main ducts 9 can be sent respectively to two distinct straws or cannulas 18, each of which can be directed to a respective nostril of the user's nose.

After exposing the user to the desired tests based on the administration of one or more desired odours, the device 1 can be turned off.

If it is desired to modify the odorous substance sent to a given peripheral duct 20, in order to avoid contamination of such new odorous substance with any residues of the odorous substance previously passing through such peripheral duct 20, which tend to accumulate particularly in the non-return valve 31 of the tubular cartridge 21 associated with such peripheral duct 20, it is possible to completely remove such tubular cartridge 21 and replace it with a new one, which is very quick and simple, as the tubular cartridges 21 are accessible from outside the delivery manifold 7.

In advantageous embodiments in which the delivery manifold 7 is provided with the one or more tubular inserts 33, in cases where it is desired to replace one or more odours to be sent to the peripheral ducts 20, in order to further reduce the risk of possible contamination with previously used odorous substances, it is further possible, besides replacing the respective tubular cartridges 21, to also remove and replace (or reposition after proper cleaning) the one or more tubular inserts 33.

From the above description, the features and advantages of the finding are clear.

In particular, due to the tubular cartridges, internally provided with a non-return valve and accessible from outside the delivery manifold for removal and repositioning, it is possible to achieve a quick and easy removal, and quick and easy installation, of the non-return valves that connect the containers of the odorous substances to the delivery manifold.

Furthermore, the device and the delivery manifold for such device, according to the finding, are suitable for easy use both in the field of basic research and in clinical or commercial settings (for example, by sommeliers, in the coffee sector, etc.).

Furthermore, in the advantageous embodiment in which the manifold is provided with one or more tubular inserts, the possibility of removing and replacing them further reduces the risk of possible contamination with previously used odorous substances, in the event that the delivery manifold is to be used for the delivery of new and different odorous substances.

Moreover, the advantageous embodiment in which the inlets of the peripheral ducts are arranged on the central body at a plurality of overlapping levels, and the inlets of the peripheral ducts on one such levels are placed side by side and staggered with respect to the peripheral ducts of a contiguous level, allows to have a high number of peripheral ducts contiguous to each other, thereby increasing the number of odours that can be simultaneously sent to the delivery manifold, and consequently allowing the device according to the finding to deliver a greater number of different odour combinations.

Moreover, the advantageous embodiment in which the one or more main ducts extend parallel to a rectilinear longitudinal direction, and each of the peripheral ducts extends along a direction inclined with respect to such rectilinear longitudinal direction, ensures that, in the case where two peripheral ducts open into the internal lateral surface of the same main duct in diametrically opposite positions, thus facing each other, the odorous airflow exiting one of such peripheral ducts is not directed towards the peripheral duct diametrically opposite to it, and therefore does not enter it, which would otherwise hinder the release of air from it or contaminate it.

Moreover, the advantageous embodiment in which the delivery manifold comprises the flow deviating means ensures that the main air flow from the second pumping or blowing means flows along the internal lateral surface of the one or more main ducts, effectively removing therefrom the odorous substances present thereon.

Finally, it is clear that the device and the delivery manifold for such device, object of the present finding, are susceptible to numerous modifications and variants, all falling within the finding; furthermore, all details are replaceable by technically equivalent elements. In practice, the materials used, as well as the size, can be any depending on the technical requirements.

## Claims

1. Device (1) for the controlled delivery of odours comprising:
- a plurality of containers (3) inside which an odorous substance can be deposited;
- first pumping or blowing means (4) configured to selectively introduce pressurized air into said containers (3) and to cause then the release of such air from said respective containers (3);
- a delivery manifold (7) comprising a central body (8) crossed by one or more main ducts (9) an inlet end (10) of which is in fluid communication with second air pumping or blowing means (11) and an outlet end (12) of which is in fluid communication with means for delivering (13) air outside said device (1);
wherein said central body (8) has a plurality of peripheral ducts (20) each of which puts one of said one or more main ducts (9) in fluid communication with the outside of said central body (8);
- electronic control means (100) configured to control the electrical and electronic components of said device (1);
**characterised in that**
said delivery manifold (7) comprises a plurality of tubular cartridges (21) provided with a transit duct (22) which passes through them from a suction end (23) of them to a delivery end (24) of them, wherein said delivery end (24) of each of said tubular cartridges (21) is fixed or fixable in a hermetic way, in a removable manner, to one of said peripheral ducts (20) so as to put the respective transit duct (22) in fluid communication with said peripheral duct (20), wherein said transit duct (22) of said tubular cartridges (21) is hydraulically connected or connectable, in correspondence of said suction end (23), to a pipe (26) hydraulically connected or connectable to one of said containers (3), wherein each of said tubular cartridges (21) contains a non-return valve (31) positioned in said transit duct (22) to prevent the passage of a fluid, through said transit duct (22), from said delivery end (24) to said suction end (23).

2. Device (1), as in claim 1, wherein said tubular cartridges (21) have an elongated shape and at least one main section (27) with a truncated conical, or cylindrical, or prismatic, or truncated pyramidal shape.

3. Device (1), as in a previous claim, wherein said one or more main ducts (9) extend parallel to a rectilinear longitudinal direction and each of said peripheral ducts (20) extends according to a direction inclined with respect to said rectilinear longitudinal direction.

4. Device (1), as in a previous claim, wherein the inlets of said peripheral ducts (20) are arranged on said central body (8) according to a plurality of overlapping levels.

5. Device (1), as in claim 4, wherein the inlets of said peripheral ducts (20) of one of said levels are placed side by side and staggered with respect to the inlets of the peripheral ducts (20) of a contiguous level.

6. Device (1), as in a previous claim, wherein said one or more main ducts (9) have a cylindrical or truncated cone or truncated pyramid or prismatic shape.

7. Device (1), as in a previous claim, wherein said central body (8) comprises a single main duct (9).

8. Device (1), as in claim 1 to 6, wherein said central body (8) comprises two main ducts (9), separate and parallel to each other.

9. Device (1), as in a previous claim, wherein said central body (8) comprises a tubular body (80) in which one or more main through holes (90) are obtained, from which a plurality of first peripheral through holes (200) branch off.

10. Device (1), as in claim 9, wherein said one or more main through holes (90) coincide with said one or more main ducts (9) and said first peripheral through holes (200) coincide with said peripheral ducts (20).

11. Device (1), as in claim 9, wherein said central body (8) comprises one or more tubular inserts (33) whose external lateral surface (34) is at least partially counter-shaped to the internal lateral surface (90a) of one of said one or more main through holes (90),
wherein said one or more tubular inserts (33) are removably inserted into said respective one or more main through holes (90) for the entire longitudinal extension of the latter,
wherein said one or more main ducts (9) are obtained in one or more of said one or more tubular inserts (33), wherein said one or more tubular inserts (33) comprise a plurality of second peripheral through holes (36) each of which branches off from one of said one or more main ducts (9) and faces a respective first peripheral through hole (200), defining, together with the latter, one of said peripheral ducts (20).

12. Device (1), as in claim 11, wherein said one or more tubular inserts (33) are fixed or fixable in said main duct (9) by means of removable coupling means.

13. Device (1), as in one or more of the preceding claims, wherein said delivery manifold (7) comprises flow deviating means (38) associated with said one or more main ducts (9) to direct towards the internal lateral surface (9a) of the same a respective main air flow (14) which enters said one or more main ducts (9) through said inlet end (10).

14. Delivery manifold (7) for an odour dispensing device (1) comprising:
- a central body (8) crossed by one or more main ducts (9);
- a plurality of peripheral ducts (20) each of which puts one of said one or more main ducts (9) in fluid communication with the outside of said central body (8);
**characterised by** the fact that it comprises:
- a plurality of tubular cartridges (21) provided with a transit duct (22) which passes through them from a suction end (23) of them to a delivery end (24) of them, wherein said delivery end (24) of each of said tubular cartridges (21) is fixed or fixable in a hermetic way, in a removable manner, to one of said peripheral ducts (20) so as to put the respective transit duct (22) in fluid communication with said peripheral duct (20), wherein each of said tubular cartridges (21) contains a non-return valve (31) positioned in said transit duct (22) to prevent the passage of a fluid, through said transit duct (22), from said delivery end (24) to said suction end (23).
